# EUROPÄISCHE PATENTSCHRIFT

(11) **EP 0 592 897 B1**
(45) Veröffentlichungstag und Bekanntmachung des Hinweises auf die Patenterteilung: **27.05.1998**
(21) Anmeldenummer: 93115908.1
(22) Anmeldetag: 01.10.1993
(51) Int. Cl.: A61F 2/30, A61F 2/32, A61F 2/36

(54) **Modularer Hüftprothesenschaft**
Modular shaft for a hip prothesis
Modulaire pour une prothèse de la hanche

(30) Priorität: 13.10.1992 CH 3193/92
(43) Veröffentlichungstag der Anmeldung: 20.04.1994
(73) Patentinhaber: Kropf, Philipp Rolf, CH-8142 Uitikon (CH); Geisser, Albert, CH-6373 Ennetbürgen (CH)
(72) Erfinder: Kropf, Philipp Rolf, CH-8142 Uitikon (CH); Geisser, Albert, CH-6373 Ennetbürgen (CH)
(74) Vertreter: Blum, Rudolf Emil Ernst

(56) Entgegenhaltungen:
- EP-A- 0 243 298
- US-A- 5 080 685
- US-A- 5 108 452

## Beschreibung

Die Erfindung betrifft einen modularen Hüftprothesenschaft gemäss dem Oberbegriff von Anspruch 1.

Derartige Schaftprothesen werden in verschiedensten Formen und Grössen hergestellt, sodass sie dem jeweils vorliegenden Knochenverlauf und Knochenzustand des Oberschenkelknochens anpassbar sind. Insbesondere besteht zum Beispiel das Bedürfnis, die Länge und Form des distalen Teils und die Dicke und Form des proximalen Teils möglichst frei zu wählen. Auch die Oberflächenbeschaffenheit der Prothese sollte variierbar sein und insbesondere darauf abgestimmt werden können, ob im proximalen und/oder im distalen Bereich Knochenzement zur Anwendung kommt.

Um eine gute Anpassbarkeit der Prothese zu gewährleisten stehen verschiedene modulare Systeme zur Verfügung, wie sie zum Beispiel in CH-671 689 oder in WO-88/01854 beschrieben werden. Diese Systeme bestehen in der Regel aus einem Modul für den proximalen Bereich und einem oder zwei Modulen für den intermediären und distalen Bereich, wobei diese Module beliebig kombinierbar sind.

Da vor allem der proximale Teil sehr vielfältig geformt sein kann, ist es selbst bei der Verwendung dieser modularen Systemen notwendig, dass dem Chirurgen eine grosse Auswahl von verschiedenen Prothesen bzw. proximalen Modulen zur Verfügung steht. Dies führt zu entsprechend hohen Herstellungs- und Anschaffungskosten.

Ausserdem müssen bestehende modulare Systeme mindestens teilweise vor der Operation zusammengestellt werden und können intraoperativ im Knochen (in situ) in ihrer Form nur beschränkt geändert werden.

In EP 243 298 und US 5 080 685 werden modulare Systeme beschrieben, bei denen ein proximaler Teil und gegebenenfalls Verlängerungsteile auf einem distalen Teil befestigt werden können.

Beim Einbringen der bekannten Prothesen in den Hüftknochen muss in der Regel zuerst Hüftknochenmaterial abgetragen werden, um eine Kavität zur Aufnahme der Prothese zu erhalten. Dies geschieht dadurch, dass der Knochen mit geeignet geformten Raspeln oder Bohrern bearbeitet wird.

Dieses Abtragen von Knochenmaterial bewirkt oftmals Probleme, da es zu einer Schwächung der Knochenstruktur führt. Auch ist es sehr zeitaufwendig und stellt an den Chirurgen hohe Anforderungen, da fehlerhaft abgetragenes Material verloren ist. Ausserdem erfordert es einen speziell angepassten Satz von entsprechenden abrasiven Bearbeitungswerkzeugen, die an die Form der zur Verfügung stehenden Modulteile angepasst sind.

Um ein Bearbeiten des Hüftknochens zumindest im distalen Teil zu umgehen, werden deshalb auch Prothesen verwendet, die im distalen Bereich aus einer dünnen Stange oder aus einem oder mehreren Drähten bestehen, sodass sie direkt ins weiche Knochenmaterial des Röhrenknochens eingetrieben werden können, ohne dass dort eine Kavität vorbereitet werden muss.

Solche Systeme zeichnen sich aber durch einen schlechen Halt aus, da sie im distalen Bereich nur im weichen Knochenmaterial gelagert sind. Damit fehlt auch eine Kraftbelastung der Kortikalis, welche zu einer Stimulierung des Knochenwachstums führen würde.

Aus diesem Grunde stellt sich die Aufgabe, einen modularen Hüftprothesenschaft bereitzustellen, der diese Nachteile mindestens teilweise nicht aufweist und vollständig intraoperativ in situ zusammengestellt werden kann.

Diese Aufgabe wird durch den im kennzeichnenden Teil des ersten Patentanspruchs beschriebenen Hüftprothesenschaft erfüllt.

Der so aufgebaute Hüftprothesenschaft ist vollständig intraoperativ aufbaubar und die einzelnen Modulteile können nacheinander vom proximalen Ende her eingebracht werden. Damit wird das Einsetzen der Prothese stark vereinfacht und Korrekturen sind im Verlauf der Operation ohne weiteres möglich. Insbesondere ist es möglich, die Modulteile intraoperativ auszuwählen und auszuprobieren.

Dank der vorgesehenen Befestigungsmittel kann der Trägerteil z.B. mittels eines oszillatorischen Einschlaginstruments auch in einen nicht vorbereiteten Knochen sicher eingeführt werden, wobei die Nut und die Abflachungen eine sichere Führung gewährleisten.

Da der Hüftprothesenschaft im proximalen Teil aus mehreren Modulen aufgebaut ist, können insbesondere die beiden wichtigsten Schaftparameter in diesem Bereich (Schaftdicke des metaphysären Teils sowie Montagewinkel und Abstand des Gelenkkopfes) durch geeignete Wahl der Module getrennt festgelegt werden.

Dank der vorzugsweisen Ausformung des Trägerteils des Hüftprothesenschaftes kann dieser in den distalen Bereich eingetrieben werden, ohne dass ein vorheriges Raspeln oder Ausbohren des Röhrenknochens notwendig wäre. Trotzdem ist ein kraftschlüssiger Kontakt mit der Kortikalis gewährleistet. Damit wird das Knochenwachstum stimuliert, was zu einer innigen Verbindung zwischen dem Prothesenschaft und dem Knochen führt.

Der Trägerteil eignet sich insbesondere dazu, dass er mit einem oszillatorischen Einschlaginstrument in den Knochen eingetrieben wird, wie es z.B. in der europäischen Patentanmeldung EP-452 543 beschrieben wird. Durch die erfindungsgemäße Ausformung des proximalen Endes des Trägerteils wird eine zug-, stoss- und drehfeste Verbindung mit dem Einschlaginstrument gewährleistet. Dies führt zu verbesserter Kontrolle der Schaftposition während des Einschlagvorgangs.

Der Trägerteil kann ohne weiteres sehr lang ausgeführt werden, was es erlaubt, den erfindungsgemässen Hüftprothesenschaft sehr weit einzutreiben. Dies ist vor allem bei Sekundärinstallationen von Vorteil, wo die Entfernung eines älteren Schaftes zu Beschädigungen im oberen oder mittleren Bereich des Röhrenknochens geführt hat. Auch wirkt ein derart langer Trägerteil ähnlich wie ein Marknagel und trägt zur Stärkung des Femurknochens bei.

Weitere Vorteile des erfindungsgemässen Hüftprothesenschafts werden aus der folgenden Beschreibung eines Ausführungsbeispiels anhand der Figuren ersichtlich. Dabei zeigen:
Figur 1 einen Schnitt durch den gesamten Hüftprothesenschaft;
Figur 2 eine Ansicht des Trägerteils; und
Figur 3 den metaphysären und den cervikalen Teil sowie deren Verbindungselemente.

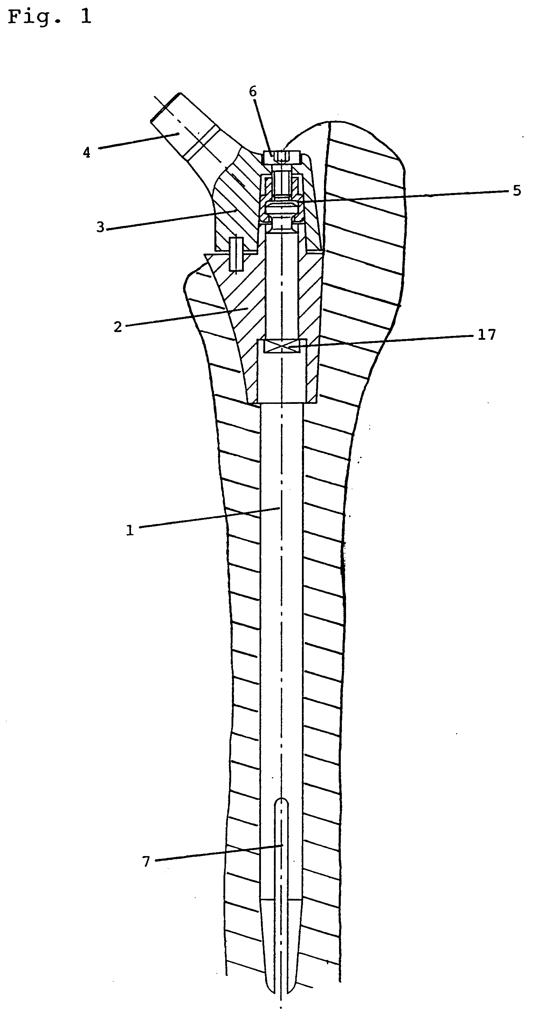

Der Gesamtaufbau eines Ausführungsbeispiels des erfindungsgemässen Hüftprothesenschaftes wird in Figur 1 gezeigt. Im wesentlichen besteht der Schaft aus drei Elementen, dem Trägerteil 1, dem metaphysären Teil 2 und dem cervikalen Teil 3.

Der Trägerteil 1 erstreckt sich dabei fast über die ganze Länge der Prothese, wobei sein unterer Bereich ihren distalen Teil bildet.

Der metaphysäre Teil 2 ist auf dem Trägerteil 1 vom proximalen Ende her aufgesteckt. Er definiert im wesentlichen die Form und den Durchmesser der Prothese im proximalen Knochenbereich.

Der cervikale Teil 3 ist auf dem metaphysären Teil 2 aufgesetzt und mittels eines Kupplungsteils 5 und einer Schraube 6 zugfest mit dem Trägerteil 1 verbunden. Zur Aufnahme eines (nicht gezeigten) Gelenkkopfs weist er einen in bekannter Weise geformten, schräg abstehenden Hals/Konus 4 auf. Durch die Länge des Halses sowie dessen Lage und Winkel zur Prothesenachse wird die Position des Gelenkkopfes definiert.

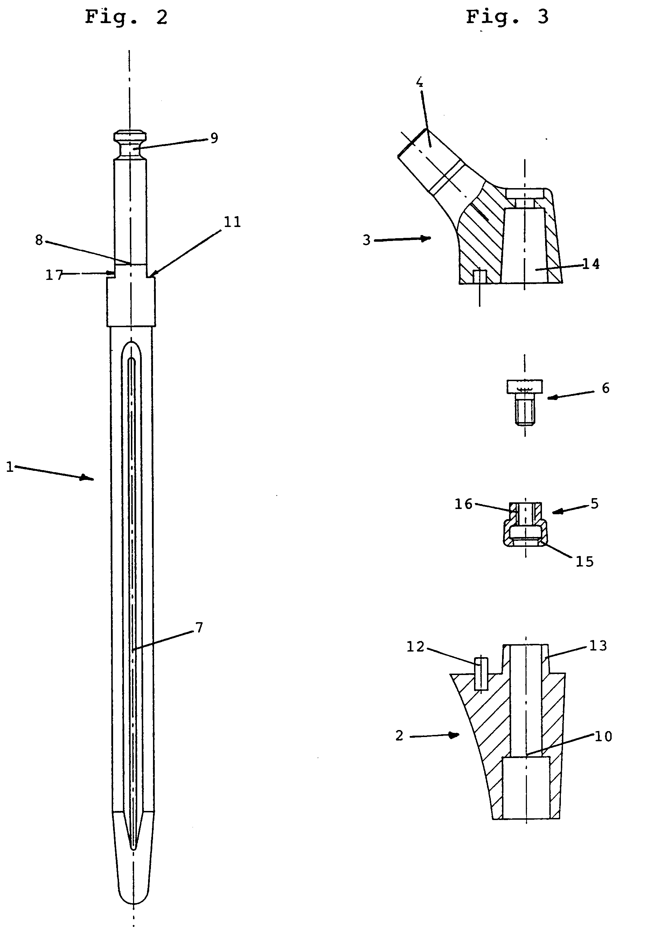

Die Figuren 2 und 3 zeigen die Teile des vorliegenden Ausführungsbeispiels der Erfindung im einzelnen.

Figur 2 zeigt eine Ansicht des Trägerteils 1, welche zu jener in Figur 1 um 90° um die Längachse gedreht ist. Dabei ist ersichtlich, dass der Trägerteil im wesentlichen in einen oberen, proximalen Kopf 8 und einen distalen Bereich aufgeteilt ist. Der distale Bereich ist am unteren Ende leicht zugespitzt und weist ansonsten eine konstante oder langsam ändernde Dicke auf, die im wesentlichen dem Innendurchmesser der Kortikalis entspricht. Je nach Knochengrösse und -zustand liegt diese Dicke in der vorliegenden Ausführung im Bereich von 7 bis 23 mm. Ausserdem kann der Trägerteil 1 in seinem distalen Bereich eine Längsnut 7 aufweisen. Diese Längsnut bildet einen Abflusskanal für das Knochenmark beim Eintreiben des Trägerteils. Damit wird es möglich, den Trägerteil direkt in den nicht vorgebohrten Röhrenknochen einzutreiben.

An seinem proximalen Ende ist der Trägerteil 1 zur zug- und stossfesten Verbindung mit einem Einschlaginstrument ausgeformt. Dazu weist er eine umgehende Nut 9 auf, in welche das Einschlaginstrument eingerastet werden kann. Ausserdem weist der Kopf 8 abgeflachte Backen 17 auf, auf welchen entsprechende Flächen des Einschlaginstruments zu liegen kommen. Damit wird der Trägerteil drehfest mit dem Einschlaginstrument verbunden. Dies ermöglicht eine gute Kontrolle der Orientierung des Trägerteils während des Einschlagens.

Der metaphysäre Teil 2 ist in Figur 3 gezeigt. Wie bereits erwähnt, definiert er die Prothesenform im proximalen Knochenbereich. Dazu besitzt er eine im wesentlichen bekannte, nach unten verjüngte Form.

Er weist eine durchgehende Oeffnung 10 auf, welche der Form des Kopfes 8 des Trägerteils 1 angepasst ist. Er wird vom proximalen Ende auf den Trägerteil 1 aufgesteckt und ruht auf dessen Schulter 11.

Je nach Ausführung kann der metaphysäre Teil 2 drehfest oder nicht drehfest mit dem Trägerteil verbunden sein. Für eine drehfeste Verbindung können z.B. die Schulter 11 sowie die Oeffnung 10 mit Kerben oder einem Raster versehen werden.

Auf den metaphysären Teil 2 wird der cervikale Teil 3 aufgesetzt. Durch geeignete Position und Länge seines Hals-/Konusteils 4 definiert dieser den Montagewinkel und -abstand des Gelenkkopfes.

Der cervikale Teil weist zur Aufnahme des leicht konischen Halses 13 des metaphysären Teils eine entsprechend angepasste Bohrung 14 auf. Zusammen mit den unten beschriebenen Befestigungselementen 5 und 6 ergibt sich somit eine kraftschlüssige Verbindung zwischen den beiden Teilen 2 und 3.

Zur Rotationssicherung zwischen den beiden Teilen 2 und 3 dient ein Stift 12.

Zur zugfesten Verbindung des cervikalen Teils 3 mit dem Trägerteil 1 dienen der Kupplungsteil 5 und die Schraube 6.

Der Kupplungsteil 5 ist derart geformt, dass er über das proximale Ende des Trägerteils 1 geschoben und mit Federarmen oder einem federnden Kragen 15 in der Nut 9 eingerastet werden kann.

Im montierten Zustand des Hüftprothesenschaftes wird der Kupplungsteil 5 vom unteren Bereich der Oeffnung 14 des cervikalen Teils 3 umschlossen. Damit wird ein Ausklinken der Federarme bzw. des Kragens 15 verhindert.

Im oberen Bereich des Kupplungsteils 5 befindet sich ein Gewinde 16, in welches vom proximalen Ende durch den cervikalen Teil die Befestigungsschraube 6 eingedreht werden kann.

Die Schraube 6 und der Kupplungsteil 5 bilden somit eine zugfeste Verbindung zwischen dem cervikalen Teil 3 und dem Trägerteil 1, dank welcher auch der metaphysäre Teil 2 fest auf den Trägerteil 1 gespannt wird.

Wie bereits erwähnt wurde, wird beim Einsetzen des erfindungsgemässen Hüftprothesenschafts mit Vorteil zuerst nur der Trägerteil 1 in den Röhrenknochen eingetrieben. Dabei braucht der Knochen nicht besonders vorbereitet zu werden, insbesondere ist ein Vorbohren nicht notwendig, wie bereits vorstehend erläutert. Die Verwendung des erwähnten hochfrequent oszillierenden Einschlaginstruments gewährleistet dabei, dass keine Kraftspitzen auftreten, welche zu einer Beschädigung des Knochens, wie z.B. einer Sprengung der Kortikalis, führen könnten.

In einem nächsten Schritt wird der metaphysäre Teil 2 auf das Kopfende 8 des Trägerteils aufgesetzt. Je nach Knochenzustand und Form des metaphysären Teils kann es dabei gegebenenfalls von Vorteil sein, den Knochen in diesem Bereich vor Einbringen des Trägerteils 1 mindestens teilweise vorzuraspeln. Allerdings kann auf das Vorraspeln unter Umständen auch verzichtet werden, insbesondere wenn der metaphysäre Teil mittels des beschriebenen oszillierenden Einschlaggeräts eingebracht wird. Damit wird die Beschädigung von proximalem Knochenmaterial minimal gehalten und eine optimale, kraftübertragende Verbindung zwischen Prothese und Knochen gewährleistet. Die resultierende homogen verteilte Belastung des Knochens fördert das Knochenwachstum.

Zum Einschlagen des metaphysären Teils 2 mit einem Einschlaggerät kann gegebenenfalls auch ein geeigneter Adapter zur Verbindung mit dem Einschlaggerät verwendet werden. Dazu kann z.B. der Hals 13 des metaphysären Teils ein Aussengewinde aufweisen, auf welches der Adapter (oder das Einschlaggerät) aufschraubbar ist.

Nach Einbringen des Trägerteils 1 und des metaphysären Teils 2 wird der Kupplungsteil 5 aufgesetzt. Auf diesen wird der cervikale Teil 3 geschoben. Damit ist der Kupplungsteil 5 gegen Abrutschen vom Trägerteil 1 gesichert.

Am Schluss wird die Verbindungsschraube 6 vom proximalen Ende her eingeschraubt. Damit werden alle Teile der Prothese miteinander verbunden.

Es ist selbstverständlich aber auch möglich, eine fertig zusammengestellte erfindungsgemässe Prothese auf diese Art direkt einzubringen. Dazu ist es aber im vorliegenden Ausführungsbeispiel von Vorteil, zur kraftschlüssigen Verbindung der Prothese mit dem Einschlagwerkzeug einen geeigneten Adapter zu verwenden. Der Adapter kann dabei z.B. anstelle der Schraube 6 eingesetzt werden und an seinem unteren Ende ein Gewinde aufweisen, welches im Kupplungsteil 5 eingeschraubt werden kann. Der obere Teil des Adapters kann z.B. wie der Kopfteil 8 des Trägerteils 1 geformt sein.

Es ist auch möglich, einen nur teilweise montierten Hüftprothesenschaft in den Knochen einzubringen. Mittels eines geeigneten Adapters können z.B. der Trägerteil 1, der metaphysäre Teil 2 und der Kupplungsteil 5 gleichzeigig eingetrieben werden. In diesem Falle könnte der Adapter z.B. an seinem unteren Ende den Hals 13 des metaphysären Teils umschliessen, gegebenenfalls gegen Rotation gesichert und mittels eines zentralen Gewindes im Kupplungsteil 5 eingeschraubt sein.

Natürlich ist die obenbeschriebene Prothese nicht die einzige Möglichkeit zur Ausführung der Erfindung. Einige weitere der möglichen Ausführungsbeispiele werden im folgenden kurz angedeutet.

Der Trägerteil 1 wurde in den Figuren 1 bis 3 gerade gezeichnet. Es ist allerdings durchaus sinnvoll, Trägerteile zur Verfügung zu stellen, welche in ihrem distalen Bereich eine der Femurform angepasste Krümmung aufweisen.

Der Abflusskanal 7 für Knochenmark kann insbesondere dann entfallen, wenn die Prothese in einen vorgebohrten oder hohlen Röhrenknochen eingetrieben wird. Dies kann z.B. auch bei der Verwendung von Knochenzement im distalen Bereich angebracht sein.

Der Trägerteil kann auch hohl ausgeführt und mit Löchern versehen werden. Ein derartiger Trägerteil erlaubt das Durchbauen des Trägerteils mit Knochenmaterial. Ausserdem hat ein hohl ausgeführter Trägerteil besser dem Knochen angepasste elastische Eigenschaften.

Anstelle von nur zwei Modulteilen 2 und 3 im proximalen Bereich können auch mehr als zwei Modulteile vorgesehen sein. In einer bevorzugten Ausführung wird z.B. der cervikale Teil 2 in einen unteren und einen oberen Teil aufgetrennt. Damit wird eine noch differenziertere Anpassung an die vorliegenden Verhältnisse möglich.

Bei Tumoren oder schwierigen Revisionen ist es manchmal nötig, den proximalen Teil des Femurknochens abzuschneiden. Dieser Knochenteil muss durch eine geeignete Prothese ersetzt werden, welche distal verankert wird. Für einen solchen Fall ist es sinnvoll, den metaphysären Teil zylindrisch oder in Knochenform auszugestalten, so dass er als proximaler Knochenersatz dienen kann.

Die Ausführung des Kopfes 8 und der Befestigung der Modulteile 2 und 3 auf dem Trägerteil 1 kann ebenfalls in verschiedenster Weise ausgeführt werden. So kann z.B. der Kopf 8 nach Einbringen des Trägerteils 1 abmontierbar sein, indem er z.B. im Trägerteil eingeschraubt ist. Die so freiwerdende Gewindeöffnung im Trägerteil kann zur Verschraubung des metaphysären und des cervikalen Teils mittels einer geeigneten Langschraube verwendet werden, welche anstelle der Schraube 6 und des Kupplungselementes 5 verwendet wird.

In einer weiteren Ausführung wird die Verbindung der Modulteile 2 und 3 und des Trägerteils 1 im wesentlichen mittels einer leicht konusförmigen Ausgestaltung des Kopfes 8 des Trägerteils 1 und des Halses 13 des metaphysären Teils 2 erreicht. Dabei wird über die Konusform der Passteile eine Verkeilung der Komponenten bewirkt, insbesondere wenn sie unter Wirkung einer axialen Kraft aufeinanderbegracht werden. Zur Sicherung kann auch in diesem Falle eine achsiale Sicherungsschraube vorgesehen sein.

## Patentansprüche

1. Modularer Hüftprothesenschaft, der einen in den Knochen eintreibbaren, den distalen Bereich des Hüftprothesenschaftes bildenden Trägerteil (1) sowie mehrere proximale Modulteile (2, 3) aufweist, wobei die proximalen Modulteile (2, 3) vom proximalen Ende aufsteckbar und kraftschlüssig mit dem Trägerteil (1) verbindbar sind, und wobei das proximale Ende (8) des Trägerteils Befestigungsmittel (9,17) geeignet zur Zug-, stoss- und drehfesten Verbindung mit einem Einschlaginstrument aufweist, dadurch gekennzeichnet, dass die Befestigungsmittel eine um den Trägerteil (1) umlaufende Nut (9) und am Trägerteil (1) ausgeformte Abflachungen (17) zur zug-, stoss- und drehfesten Verbindung mit dem Einschlaginstrument umfassen.

2. Hüftprothesenschaft nach Anspruch 1, dadurch gekennzeichnet, dass der Trägerteil (1) im distalen Bereich und mindestens über einen Teil seiner Länge mindestens eine längs verlaufende Ausnehmung (7) zur Ableitung von Knochenmark während des Einbringvorgangs aufweist.

3. Hüftprothesenschaft nach einem der vorhergehenden Ansprüche, dadurch gekennzeichnet, dass einer der proximalen Modulteile (2, 3) ein metaphysärer Teil (2) ist, dessen Durchmesser zum proximalen Ende hin zunimmt.

4. Hüftprothesenschaft nach Anspruch 3, dadurch gekennzeichnet, dass zur drehfesten Verbindung des metaphysären Teils (2) mit dem Trägerteil (1) eine Schulter (11) des Trägerteils (1) und eine Öffnung (10) des metaphysären Teils (2) mit Kerben oder einem Raster versehen sind.

5. Hüftprothesenschaft nach einem der vorhergehenden Ansprüche, dadurch gekennzeichnet, dass einer der proximalen Modulteile (2, 3) ein cervicaler Teil (3) ist, welcher einen seitlich abstehenden Hals (4) zur Aufnahme eines Gelenkkopfs aufweist.

6. Hüftprothesenschaft nach einem der Ansprüche 3 oder 4 und nach Anspruch 5, dadurch gekennzeichnet, dass der cervicale Teil (3) mit einem Stift (12) drehfest mit dem proximalen Ende des metaphysären Teils (2) verbunden ist.

7. Hüftprothesenschaft nach den Ansprüchen 3 und 5 oder nach Anspruch 6, dadurch gekennzeichnet, dass der cervicale Teil (3) am proximalen Ende des metaphysären Teils (2) anschliesst und eine zugfeste Verbindung (5, 6) mit dem Trägerteil (1) aufweist.

8. Hüftprothesenschaft nach einem der vorhergehenden Ansprüche, dadurch gekennzeichnet, dass der Trägerteil (1) in seinem distalen Bereich eine Krümmung aufweist.

9. Hüftprothesenschaft nach einem der vorhergehenden Ansprüche, dadurch gekennzeichnet, dass der Trägerteil (1) in seinem distalen Bereich im wesentlichen einen Durchmesser zwischen 7 mm und 23 mm aufweist.

## Claims

1. A modular shaft for a hip prosthesis having a stem (1) forming the distal part of said shaft for the hip prosthesis, whereby said stem could be driven into the bone, as well as a plurality of modular proximal parts (2, 3), whereby said modular proximal parts (2, 3) are attachable and frictionally connected to said stem (1) from the proximal end, and whereby the proximal end (8) of said stem comprises fasteners (9, 17) being suitable for a connection transferring pulling, pushing and rotating forces to a percussion tool, characterised in that the fasteners comprise a circumferential groove (9) around said stem, and that said stem (1) comprises flat lateral faces (17), for the rigid connection with the percussion tool.

2. The modular shaft for a hip prosthesis according to claim 1, characterised in that said stem (1) has at least one recess (7) for draining medullar material while driving said stem into a bone, said recess extending over a distal section of said stem and at least one part of the length of said stem.

3. The modular shaft for a hip prosthesis according to one of the preceding claims, characterised in that one of said modular proximal parts (2, 3) is a metaphysary part (2) with a diameter that is increasing towards a proximal end of said metaphysary part.

4. The modular shaft for a hip prosthesis according to claim 3, characterised in that for the rigid connection of the metaphysary part (2) with the stem (1), a shoulder (11) of said stem (1) and an opening of the metaphysary part (2) with notches or with an interlocking profile is provided.

5. The modular shaft for a hip prosthesis according to one of the preceding claims, characterised in that one of said modular proximal parts (2, 3) is a cervical part (3) having a laterally projecting neck (4) for receiving a head of the hip joint.

6. The modular shaft for a hip prosthesis according to one of the claims 3 or 4 and according to claim 5, characterised in that said cervical part (3) is mounted to said proximal end of said metaphysary part (2) with a peg (12) such that said cervical part is not rotatable in respect to said metaphysary part (2).

7. The modular shaft for a hip prosthesis according to one of the claims 3 and 5 or according to claim 6, characterised in that said cervical part (3) is adjacent to said proximal end of said metaphysary part (2) and has a rigid connection (5, 6) to said stem (1).

8. The modular shaft for a hip prosthesis according to one of the preceding claims, characterised in that said stem (1) has a bent in its distal section.

9. The modular shaft for a hip prosthesis according to one of the preceding claims, characterised in that said stem (1) displays in its distal section a diameter of between 7 mm and 23 mm.

## Revendications

1. Fût modulaire de prothèse de la hanche comportant une base (1) qui forme sa partie distale et est destinée à être enfoncée dans l'os, ainsi que plusieurs parties modulaires proximales (2, 3) pouvant être engagées du côté proximal sur la base (1) et réunies à force avec celle-ci, l'extrémité proximale (8) de la base comportant des moyens de fixation (9, 17) pouvant être réunis de façon résistant à la traction, à la poussée et à la torsion, avec un outil de martelage, caractérisé en ce que les moyens de fixation comportent une rainure (9) encerclant la base (1) ainsi que des rebords plats (17) formés sur la base (1) pour l'accoupler de façon résistant à la traction, à la poussée et à la rotation avec l'outil de martelage.

2. Fût de prothèse de hanche selon la revendication 1, caractérisé en ce que la base (1) comporte, à sa partie distale et au moins sur une partie de sa longueur au moins une rainure longitudinale (7) pour évacuer la moelle de l'os au cours de la pose.

3. Fût de prothèse de hanche selon une des revendications précédentes, caractérisé en ce qu'une des parties modulaires proximales (2, 3) est une partie métaphysaire (2) dont le diamètre augmente en direction de l'extrémité proximale.

4. Fût de prothèse de hanche selon la revendication 3, caractérisé en ce qu'un épaulement (11) de la base (1) et une ouverture (10) de la partie métaphysaire (2) sont pourvus d'encoches ou d'une trame pour assurer l'immobilisation en rotation de la partie métaphysaire (2) par rapport à la base (1).

5. Fût de prothèse de hanche selon une des revendications précédentes, caractérisé en ce que l'une des parties modulaires proximales (2, 3) est un organe cervical (3) possédant une protubérance (4) dépassant latéralement pour recevoir une rotule.

6. Fût de prothèse de hanche selon une des revendications 3 ou 4 et la revendication 5, caractérisé en ce que la partie cervicale (3) est fixée à l'extrémité proximale de la partie métaphysaire (2) et immobilisée en rotation par rapport à celle-ci au moyen d'une cheville (12).

7. Fût de prothèse de hanche selon les revendications 3 et 5 ou selon la revendication 6, caractérisé en ce que la partie cervicale (3) jouxte l'extrémité proximale de la partie métaphysaire (2) et est réunie à la base (1) par une fixation (5, 6) résistant à la traction.

8. Fût de prothèse de hanche selon une des revendications précédentes, caractérisé en ce que la base (1) est courbe dans sa partie distale.

9. Fût de prothèse de hanche selon une des revendications précédentes, caractérisé en ce que la partie distale de la base (1) présente un diamètre compris entre 7 mm et 23 mm.
